# EUROPEAN PATENT APPLICATION

(11) **EP 3 560 360 A1**
(43) Date of publication of application: **30.10.2019**
(21) Application number: 17884377.7
(22) Date of filing: 07.12.2017
(51) Int. Cl.: A24F 47/00

(54) **CIGARETTE CARTRIDGE, ATOMIZING ASSEMBLY AND ELECTRONIC CIGARETTE**

(30) Priority: 20.12.2016 CN 201621406879 U; 20.12.2016 CN 201621411023 U; 20.12.2016 CN 201621406878 U
(71) Applicant: Changzhou Patent Electronic Technology Co., Ltd, Jiangsu Province 213001 (CN)
(72) Inventor: QIU, Weihua, Jiangsu Province 213001 (CN)
(74) Representative: Haseltine Lake Kempner LLP
(86) International application number: PCT/CN2017/115022
(87) International publication number: WO 2018/113528

(57) **Abstract**

Provided are a cigarette cartridge (1), an atomizing assembly (30a) and an electronic cigarette having same. The cigarette cartridge (1) comprises a cigarette cartridge housing (211b), and a liquid storage chamber (101), an air inlet channel (102) and a vapor outlet channel (103) provided in the cigarette cartridge housing (21 lb), wherein the air inlet channel (102) and the vapor outlet channel (103) are provided on the same side of the liquid storage chamber (101). The air inlet channel (102) and the vapor outlet channel (103) are placed inside the cigarette cartridge (1), thereby simplifying the structure; and the air inlet channel (102) and the vapor outlet channel (103) are arranged closely side-by-side, thereby shortening the length of an air path between an air inlet (104) and a vapor outlet (110), and effectively solving the problem that a user needs to use a great amount of sucking force to inhale the vapor.

## Description

### TECHNICAL FIELD

The present invention relates to the technical filed of electronic cigarette, and in particular to a cigarette cartridge, an atomizing assembly and an electronic cigarette.

### BACKGROUND

A conventional electronic cigarette generally includes a cigarette cartridge, an atomizer and a control assembly. The atomizer typically includes a heating member, a liquid guiding member, an air inlet passage and a smoke outlet passage. The setting of the air inlet passage and the smoke outlet passage of the electronic cigarette will affect the final smoke effect, the air inlet passage and the smoke outlet passage of existing electronic cigarette are not ideally set, which affects the user's inhaling experience.

### SUMMARY

In order to solve the above problem regarding the arrangement of the air inlet passage and the smoke outlet passage, the technical solution adopted by the present invention is as follows.

The first aspect of the present invention provides a cigarette cartridge including a cartridge housing, a liquid storage chamber, an air inlet passage and a smoke outlet passage. The liquid storage chamber, the air inlet passage and the smoke outlet passage are provided in the cartridge housing. The air inlet passage and the smoke outlet passage are disposed on the same side of the liquid storage chamber.

In one embodiment, the liquid storage chamber, the air inlet passage and the smoke outlet passage are arranged closely side-by-side.

In one embodiment, the cigarette cartridge includes a top wall and a peripheral wall. The liquid storage chamber is surrounded by the top wall and the peripheral wall. The air inlet passage and the smoke outlet passage are provided in the peripheral wall and located on the same side of the cigarette cartridge.

In one embodiment, an air inlet hole is defined in the peripheral wall of the cigarette cartridge, and the air inlet hole is in communication with the air inlet passage.

In one embodiment, a partition wall is formed between the air inlet passage and the smoke outlet passage.

In one embodiment, the lower end of the smoke outlet passage is narrower than the upper end of the smoke outlet passage.

In one embodiment, an inserting member is disposed in the smoke outlet passage. After the inserting member is inserted into the smoke outlet passage, the smoke outlet passage is substantially trumpet-shaped having a wide upper end and a narrow lower end.

In one embodiment, the cigarette cartridge includes a main body, a top cover and a bottom cover. The top cover is provided with a smoke outlet hole, and the smoke outlet passage is in communication with the smoke outlet hole.

In one embodiment, the inner wall of the smoke outlet passage is provided with a slope or a bump. After the smoke outlet passage is provided with the slope or the bump, the smoke outlet passage is substantially trumpet-shaped having a wide upper end and a narrow lower end.

In one embodiment, the inserting member has a wedge-shaped upper end and a column-shaped lower end. Optionally, the inserting member is a cylinder, the outer wall of the cylinder is axially attached to the inner wall of the smoke outlet passage, and a tapered hole is defined in the inserting member and penetrates through the inserting member. The size of the tapered hole gradually decreases from top to bottom.

The second aspect of the present invention provides an atomizing assembly including any one of the cigarette cartridge according to the first aspect of the present invention. The atomizing assembly further includes an atomizer detachably secured to the bottom end of the cigarette cartridge.

In one embodiment, the atomizer includes an atomizing head. The atomizing head includes an atomizing head housing, a heating member and a liquid guiding member. The atomizing head housing includes a housing body and an atomizing head top cover. A cavity is defined inside the housing body. The heating member and the liquid guiding member are both located in the cavity. The liquid guiding member is sleeved on the outer circumference of the heating member. The atomizing head top cover is provided with a liquid inlet hole in communication with the cavity. The liquid guiding member is located under the liquid inlet hole to ensure the e-cigarette liquid flowing through the liquid inlet hole to be guided into the liquid guiding member. A blocking portion is disposed between the heating member and the liquid inlet hole. The upper end surface of the blocking portion abuts against the lower end surface of the atomizing head top cover, the lower end surface of the blocking portion abuts against the upper end surface of the liquid guiding member to prevent the e-cigarette liquid flowing through the liquid inlet hole from directly contacting the heating member without flowing through the liquid guiding member.

In one embodiment, the atomizer includes an atomizing head main body. The atomizing head main body is provided with an atomizing head mounting hole. The atomizing head is disposed in the atomizing head mounting hole.

In one embodiment, the outer peripheral wall of the housing body is provided with a limiting portion.

In one embodiment, the atomizing head mounting hole is provided with a matching portion that is fixedly coupled with the limiting portion. The atomizing head is fixed in the atomizing head mounting hole by the matching portion.

In one embodiment, the limiting portion is an L-shaped groove provided in the outer peripheral wall of the housing body. Corresponding to the position of the L-shaped groove, the inner peripheral wall of the atomizing head mounting hole is provided with a protrusion that cooperates with the L-shaped groove, and the protrusion is the matching portion. Optionally, the limiting portion is a protrusion formed on the outer peripheral wall of the housing body. Corresponding to the position of the protrusion, the inner peripheral wall of the atomizing head mounting hole is provided with an L-shaped groove that cooperates with the protrusion, and the L-shaped groove is the matching portion.

The second aspect of the present invention provides an atomizing assembly including any one of the cigarette cartridge according to the first aspect of the present invention. The atomizing assembly further includes an atomizer. The atomizer is provided with an atomizing heating passage. One end of the atomizing heating passage is in communication with the air inlet passage, and the other end is in communication with the smoke outlet passage.

In one embodiment, the atomizing head includes a liquid storage tube and an inner sleeve member inserted into the liquid storage tube. The inner sleeve member includes an atomizing tube, and the interior space of the atomizing tube is the atomizing heating passage.

In one embodiment, the atomizing head includes a heating member and a liquid guiding member. The heating member is wrapped around the liquid guiding member, or the liquid guiding member is wrapped around the heating member.

The fourth aspect of the present invention provides an electronic cigarette including any one of the atomizing assemblies according to the second aspect and the third aspect of the present invention.

Compared with the prior art, the air inlet passage and the smoke outlet passage are provided on the same side of the cigarette cartridge. The air inlet passage and the smoke outlet passage are placed inside the cigarette cartridge, thereby simplifying the originally crowded structure of the atomizer. The same side arrangement of the air inlet passage and the smoke outlet passage is compact in structure, and the air inlet passage and the smoke outlet passage are arranged closely side-by-side, thereby shortening the length of an air path between the air inlet hole and the smoke outlet hole, and effectively solving the problem that a user needs to use a great sucking force to inhale the smoke. The user's inhaling is more convenient and labor-saving. In one embodiment, the lower end of the smoke outlet passage is narrower than the upper end of the smoke outlet passage, so that a negative pressure is more easily formed in the smoke outlet passage, and the suction effect is better.

### BRIEF DESCRIPTION OF THE DRAWINGS

The accompanying drawings are included to provide a further understanding of the disclosure and are used in explaining the disclosure together with the specific embodiments below. However, it should not be construed as limiting the invention. In the drawing,
FIG. 1 is a cross-sectional view of an electronic cigarette in a first embodiment.
FIG. 2 is an exploded, perspective view of one example of the cigarette cartridge in the first embodiment.
FIG. 3 is a cross-sectional view along line A-A of one example of the cigarette cartridge in the first embodiment.
FIG. 4 is a cross-sectional view along line B-B of one example of the cigarette cartridge in the first embodiment.
FIG. 5 is a cross-sectional view of another example of the cigarette cartridge in the first embodiment.
FIG. 6 is a cross-sectional view of the electronic cigarette in a second embodiment.
FIG. 7 is a cross-sectional view of the electronic cigarette shown in FIG. 6 from another aspect.
FIG. 8 is a perspective view of the atomizing head of the electronic cigarette shown in FIG. 6.
FIG. 9 is an exploded view of the atomizing head shown in FIG. 8.
FIG. 10 is a top view of the atomizing head shown in FIG. 8.
FIG. 11 is a cross-sectional view of the atomizing head shown in FIG. 8.
FIG. 12 is a cross-sectional view of the atomizing head shown in FIG. 8 from another aspect.
FIG. 13 is a cross-sectional view of an atomizing head in a third embodiment.
FIG. 14 is a cross-sectional view showing partially the atomizing head in the third embodiment.
FIG. 15 is an exploded view of the atomizing head in the third embodiment.
FIG. 16 is a cross-sectional view of a cigarette cartridge in the third embodiment.
FIG. 17 is an exploded view of the cigarette cartridge in the third embodiment.
FIG. 18 is a cross-sectional view of the electronic cigarette in the third embodiment.

The following table list various components and reference numerals.

| | |
|---|---|
| main body 10 | bottom cover 12 |
| top cover 11 | locking portion 30 |
| mounting hole 107 | decorative strip 108 |
| smoke outlet hole 110 | partition wall 106 |
| slope 14 | sensor 6, 46b |
| electrical connecting member 4 | sealing plug 5 |
| connecting portion 60 | control assembly 7 |
| outer casing 8 | USB connection hole 80 |
| USB interface 9 | smoke guiding cover 25a |
| control assembly 40a | baffle 26a |
| button hole 11a | atomizing assembly 30a |
| USB port 12a | mounting bracket 41a |
| trigger button 13a | circuit board 42a |
| top wall 21a | power source 45a |
| peripheral wall 22a | insulating member 313a |
| receiving groove 211a | atomizing head housing 11b |
| atomizing head top cover 19b | housing body 20b |
| liquid storage chamber hole 25b | atomizing head main body 31b |
| battery assembly 30b | shell 40b |
| sealing member 23b | air inlet passage 44b |
| cartridge housing 211b | |
| plugging hole 120 | cigarette cartridge 1, 20a, 21b |
| mounting groove 105 | air inlet passage 102, 221a |
| locking hole 109 | smoke outlet passage 103, 222a, 43b |
| inserting member 13 | atomizing head 2, 31a, 10b |
| main board 70 | liquid guiding member 21, 12a, 12b |
| battery 71 | heating member 22, 315a, 13b |
| main body 3 | liquid storage chamber 101, 23a, 24b |
| recess 50 | air inlet hole 104, 24a, 42b |
| shell 10a | conductor 314a |
| liquid storage gap 316a | atomizing tube 3121a |
| smoke guiding hole 251a | liquid guiding tube 3122a |
| liquid inlet hole 261a | smoke outlet hole 3123a |
| main body 301a | liquid injecting hole 3124a |
| liquid storage tube 311a | liquid inlet opening 3125a |
| inner sleeve member 312a | liquid guiding pipe 3126a |
| liquid inlet hole 16b | limiting portion 111b |
| blocking portion 17b | atomizing head mounting hole 311b |
| smoking outlet hole 41b | air outlet hole 18b |
| circuit board 45b | atomizing head air passage 131b |
| matching portion 113b | sealing gasket 47b |

### DETAILED DESCRIPTION OF EMBODIMENTS

The specific embodiments of the present invention will be described in detail below with reference to the accompanying drawings. It is to be understood that the specific embodiments described herein are merely illustrative and not restrictive.

### First embodiment

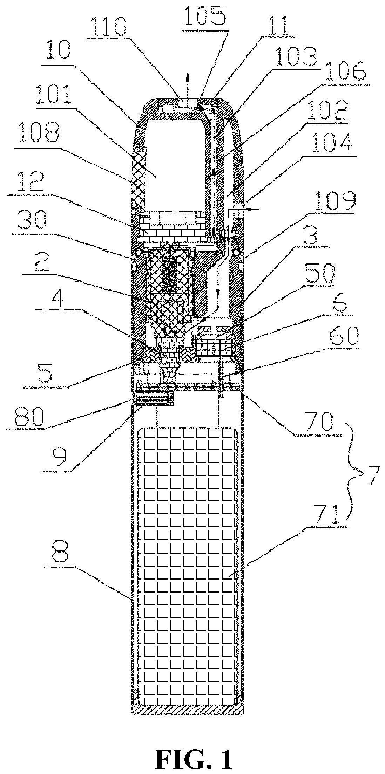

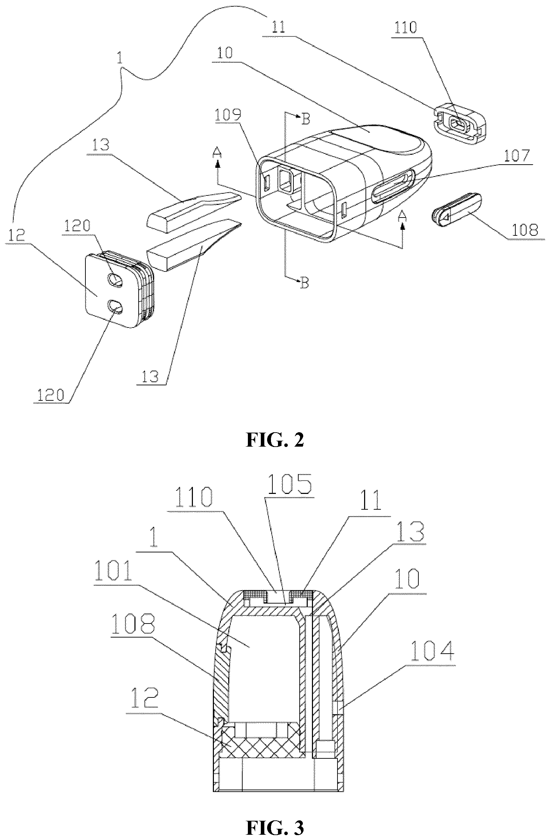

Referring to FIG. 1 and FIG. 2, the electronic cigarette of the present embodiment includes an outer casing 8, a cigarette cartridge 1 mounted on the top of the outer casing 8, an atomizing assembly (not labeled) mounted within the outer casing 8, and a control assembly 7. The cigarette cartridge 1 provides e-cigarette liquid to the atomizing assembly. The atomizing assembly atomizes the e-cigarette liquid flowed in. The control assembly 7 is mounted at the lower end of the atomizing assembly, and is electrically connected to the atomizing assembly to supply power to the atomizing assembly. A USB connection hole 80 is provided in the peripheral wall of the outer casing 8 for charging the control assembly 7 by an external power source.

The cigarette cartridge 1 includes a main body 10, a top cover 11 and a bottom cover 12. The main body 10, the top cover 11 and the bottom cover 12 constitute a cartridge housing (not shown). The top of the main body 10 defines a mounting groove 105, the top cover 11 is mounted in the mounting groove 105, and a smoke outlet hole 110 is defined at the center position of the top cover 11. The main body 10 is provided with a liquid storage chamber 101 therein. The liquid storage chamber 101 is a top closed, bottom open structure. The bottom cover 12 seals the bottom opening of the liquid storage chamber 101. In addition, an air inlet passage 102 and a smoke outlet passage 103 are formed in the axial direction inside the main body 10. The air inlet passage 102 and the smoke outlet passage 103 are disposed on the same side of the liquid storage chamber 101. Further, the liquid storage chamber 101, the air inlet passage 102 and the smoke outlet passage 103 are arranged closely side-by-side. The size of the air inlet passage 102 is the same from top to bottom, and the size of the smoke outlet passage 103 is the same from top to bottom. Optionally, the air inlet passage 102 and the smoke outlet passage 103 have a circular or square cross-section. The side wall of the main body 10 is provided with an air inlet hole 104 in communication with the air inlet passage 102. The air inlet passage 102 and the smoke outlet passage 103 are both located on the same side of the cigarette cartridge 1, and the smoke outlet passage 103 is in communication with the smoke outlet hole 110. By the above arrangement, the length of the air path from the air inlet hole 104 to the smoke outlet hole 110 is shortened, thereby effectively solving the problem that the user needs to use a great sucking force to inhale the smoke, and the user's inhaling is more convenient and labor-saving. In addition, the side wall of the liquid storage chamber 101 is provided with a mounting hole 107, a decorative strip 108 is mounted in the mounting hole 107, and the decorative strip 108 is made of transparent material. Therefore, the level of the e-cigarette liquid in the liquid storage chamber 101 can be observed through the decorative strip 108, and the amount of the liquid in the liquid storage chamber 101 can be easily checked.

Specifically, a partition wall 106 is formed between the air inlet passage 102 and the smoke outlet passage 103. The air inlet passage 102 is located adjacent to the outer peripheral surface of the main body 10, the smoke outlet passage 103 is located adjacent to the liquid storage chamber 101. The air inlet passage 102 extends axially from the bottom of the peripheral wall of the main body 10 by a predetermined height, the peripheral wall of the main body 10 is provided with an air inlet hole 104 corresponding to and in communication with the air inlet passage 102. In this embodiment, the air inlet hole 104 is in communication with the top end of the air inlet passage, such that after being supplied from the air inlet hole 104, the airflow can flow out from the bottom of the peripheral wall along the air inlet passage 102. The smoke outlet passage 103 extends axially from the bottom of the peripheral wall to the top of the peripheral wall, and is in communication with the mounting groove 105, so that the airflow flows from the bottom of the smoke outlet passage 103 and flows out from the top of the peripheral wall to the mounting groove 105, the user then inhales through the smoke outlet hole 110. Thus, this embodiment realizes air intake and smoke flowing out on the same side of the cigarette cartridge 1, the airflow enters at the upper end and flows out at the upper end, thereby solving the problem that the air inlet hole of existing electronic cigarette is opened at the bottom of the outer casing to cause the e-cigarette liquid to leak easily. In one embodiment, the air inlet passage 102 and the smoke outlet passage 103 are each formed by the inner cavity of a tubular body, the air inlet passage 102 and the smoke outlet passage 103 are arranged side-by-side. In another embodiment, the air inlet passage 102 is an inner chamber of a tubular body, the smoke outlet passage 103 is disposed around the air inlet passage 102. Further, the air inlet passage 102 is housed in the main body 10 and a part of the passage wall of the air inlet passage 102 is connected to the inner wall of the main body 10, and the other part of the passage wall is surrounded by the smoke outlet passage 103. Specifically, the space between the passage wall of the air inlet passage 102, the inner wall of the main body 10 and the wall of the liquid storage chamber 101 constitutes the smoke outlet passage 103.

Referring to FIG. 1, FIG. 5 and FIG. 6, the atomizing assembly is mounted at the bottom of the cigarette cartridge 1. The bottom of the atomizing assembly is connected to the outlet of the air inlet passage 102, and the top of the atomizing assembly is connected to the inlet of the smoke outlet passage 103. The atomizing assembly atomizes the e-cigarette liquid flowing from the cigarette cartridge 1 into the liquid storage chamber 101 to generate smoke, so that the smoke can be carried out through the airflow. The atomizing assembly includes a main body 3 and an atomizing head 2 installed in the main body 3. The main body 3 has a tubular shape and is installed in the outer casing 8. The top of the main body 3 is detachably assembled to the bottom of the cigarette cartridge 1. Specifically, the bottom surface of the main body 10 is provided with a locking hole 109, both sides of the top of the main body 3 are protruded to form a locking portion 30 respectively. Each of the locking portions 30 is correspondingly inserted into one of the locking holes 109. The provision of the locking portion 30 on the main body 3 enables the size of the locking portion 30 to be made larger, thereby effectively preventing the cigarette cartridge 1 from being detached from the atomizing assembly. The atomizing head 2 is provided with an atomizing heating passage (not shown). One end of the atomizing heating passage communicates with the air inlet passage 102, and the other end communicates with the smoke outlet passage 103. Specifically, an air gap (not shown) is disposed between the air inlet passage 102 and the atomizing head 2, and between the air smoke outlet passage 103 and the atomizing heating passage, respectively. Thereby, the air inlet passage 102 is in communication with the smoke outlet passage 103.

The structure of the atomizing head 2 in this embodiment is the same as that of the atomizing head in the third embodiment. The detailed structure is described in the third embodiment, details are not described herein again. It can be understood that in other embodiments, the atomizing head 2 can adopt other structures, and is not limited thereto.

Further, the bottom of the main body 3 is provided with a sealing plug 5. The sealing plug 5 is provided with a perforation (not labeled) corresponding to the positive electrode contact 23. An electrical connecting member 4 is arranged in the perforation. The upper end of the electrical connecting member 4 is connected to the positive electrode contact 23, the lower end of the electrical connecting member 4 protrudes from the bottom of the sealing plug 5. In addition, the sealing plug 5 is provided with a recess 50 corresponding to the air inlet passage 102, the recess 50 is in communication with the air inlet passage 102. A sensor 6 is mounted within the recess 50, the sensor 6 can be selected as an air pressure sensor or an air flow sensor. The sensor 6 projects downwardly to form a connecting portion 60 which extends beyond the bottom of the sealing plug 5.

Please referring to FIGs. 2-4, the present embodiment changes the internal structure of the smoke outlet passage 103 by narrowing the lower end of the smoke outlet passage 103 with respect to the upper end thereof, thereby optimizing the suction effect and improving the sensitivity of the sensor 6.

In one embodiment, an external member such as an inserting member 13 is inserted into the smoke outlet passage 103 to narrow the lower end of the smoke outlet passage 103 with respect to the upper end thereof.

In another embodiment, a slope or a bump is provided in the passage wall of the smoke outlet passage 103, and the lower end of the smoke outlet passage 103 is narrower than the upper end thereof by directly changing the shape of the passage wall.

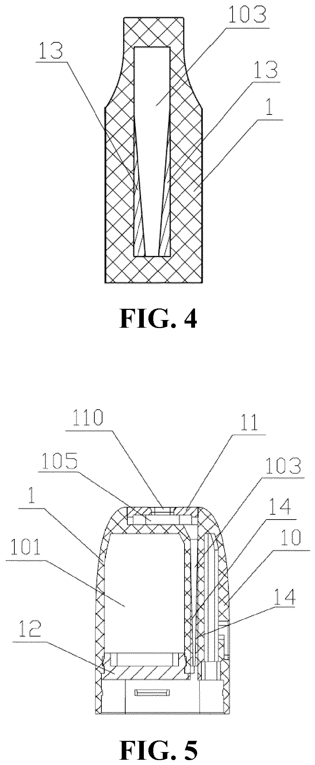

The following is a detailed description. First, please refer to FIG. 2 and FIG. 4, in this embodiment, two oppositely disposed inserting members 13 are disposed in the smoke outlet passage 103, so that the lower end of the smoke outlet passage 103 is narrower than the upper end thereof, thereby making it easier to form a negative pressure in the smoke outlet passage 103, the sensitivity of the sensor 6 is increased after the insertion of the inserting member 13. The two inserting members 13 can be selected as a silicone plug, and the opposite sides are attached on the inner wall of the smoke outlet passage 103 along the axial direction of the smoke outlet passage 103. The inserting member 13 is interference fit with the smoke outlet passage 103 to fix the inserting member 13 in the smoke outlet passage 103; or two adjacent inserting members 13 are attached to the inner wall of the smoke outlet passage 103. It can be understood that when the cross-section of the smoke outlet passage 103 is square, the inserting member 13 can be set in different directions. For example, the two inserting members 13 of FIG. 4 are disposed in the front-rear direction of the smoke outlet passage 103. In another embodiment, the inserting member 13 is disposed in the left-right direction of the smoke outlet passage 103 (the front-rear direction refers to the A-A direction in FIG. 2, and the left-right direction refers to the B-B direction in FIG. 2). Alternatively, two inserting members 13 are attached adjacent to the two inner walls of the smoke outlet passage 103. It should be noted that the lengths of the two inserting members 13 can be the same as the length of the smoke outlet passage 103, or can be shorter than the smoke outlet passage 103. After the two inserting members 13 are added, the smoke outlet passage 103 is substantially trumpet-shaped having a wide upper end and a narrow lower end. It can be understood that the two inserting members 13 can also be sealing plugs, TPUs and the like having certain deformation characteristics.

In this embodiment, the two inserting members 13 have a cylindrical shape at the lower end and a wedge shape at the upper end. Thus, when the two inserting members 13 are inserted into the smoke outlet passage 103, the upper end of the inserting member 13 having the wedge shape partially contacts the inner wall of the upper end of the smoke outlet passage 103, and the lower end of the inserting member 13 having the cylindrical shape partially contacts the inner wall of the lower end of the smoke outlet passage 103, so that a trumpet-shaped air passage having a wide upper end and a narrow lower end can be formed, which is favorable for forming a negative pressure during smoking, and the smoking effect is better.

In one embodiment, the inserting member 13 is one, the outer wall of the inserting member 13 is axially attached to the inner wall of the smoke outlet passage 103. The inserting member 13 can be selected as a cylinder, a tapered hole is defined in the inserting member 13 and penetrates through the inserting member 13. The size of the tapered hole gradually decreases from top to bottom.

In one embodiment, the inserting members 13 are three, each inserting member 13 is disposed along the axial direction of the smoke outlet passage 103, the three inserting members 13 are arranged circumferentially along the inner wall of the smoke outlet passage 103, the inserting member 13 is generally wedge-shaped with a small upper end and a large lower end. The inserting member 13 is disposed in the smoke outlet passage 103 such that the smoke outlet passage 103 has a trumpet shape with a wide upper end and a narrow lower end. In other embodiments, the inserting members 13 are four or more, and the arrangement is the same as when the inserting members 13 are three.

Referring to FIG. 5, in another embodiment, the smoke outlet passage 103 can be directly formed into a structure having a wide upper end and a narrow lower end, such that the lower end of the smoke outlet passage 103 is narrower than the upper end. This structure has the same function as the provision of the inserting member 13 in the smoke outlet passage 103. Illustratively, by providing two slopes 14 axially opposite to each other on the inner wall of the smoke outlet passage 103, optionally, the slope 14 has a wedge shape, so that the smoke outlet passage 103 is generally trumpet-shaped with a wide upper end and a narrow lower end, which is favorable for forming a negative pressure when sucking, the smoking effect is better. In addition, the slope 14 can also be a wedge-shaped bump, and is not limited thereto.

The control assembly 7 includes a main board 70 and a battery 71. The main board 70 is disposed at a lower end of the sealing plug 5. The battery 71 is located at the lower end of the main board 70 and is electrically connected to the main board 70. The lower end of the electrical connecting member 4 abuts against the upper surface of the main board 70 to make electrical contact with the main board 70. Thereby, the electrical connection between the atomizing head 2 and the main board 70 is realized. The connecting portion 60 is inserted into the hole of the main board 70, such that the sensor 6 is connected to the main board 70 to control the operation of the battery 71. When the user smokes, the sensor 6 senses the negative pressure and starts to work, causing the battery 71 to output a voltage to the main board 70, so that the atomizing head 2 operates normally. A USB interface 9 corresponding to the USB connection hole 80 is mounted on the lower surface of the main board 70 for external power supply to charge the battery 71. The sealing plug 5 can create a negative pressure sensing space for the sensor 6 inside the main body 3, and can also prevent the condensed smoke droplets from falling onto the main board 70 and damaging the main board 70. In the embodiment, the sealing plug 5 is a silicone plug. In other embodiments, the sealing plug 5 may also be a rubber plug, and is not limited thereto.

In addition, the cigarette cartridge 1, the main body 3 and the various components of the atomizing head 2 are assembled together by O-shaped silicone rings (not shown), thereby effectively preventing the leakage of the electronic cigarette. The atomizing assembly is detached from the cigarette cartridge 1 to facilitate replacement of the atomizing head 2.

When smoking, the airflow enters from the air inlet hole 104, flows into the interior of the main body 3, causes a negative pressure in the main body 3, and then the sensor 6 senses a negative pressure, causing the main board 70 and the atomizing head 2 to operate; meanwhile, the airflow enters the inside of the atomizing head 2 from the bottom of the atomizing head 2, the smoke is taken out from the top of the atomizing head 2 to the smoke outlet passage 103, and finally flows to the smoke outlet hole 110, to complete a suck.

In summary, the cigarette cartridge, the atomizing assembly and the electronic cigarette of the embodiment have the following beneficial effects:
1. When smoking, the airflow enters from the outer periphery of the cigarette cartridge 1 and passes through the atomizing head 2, the airflow enters the bottom of the atomizing head 2 firstly, then exits from the top of the atomizing head 2, and finally exits through the top of the cigarette cartridge 1, to complete a suck. The air inlet passage 102 and the smoke outlet passage 103 are disposed on the same side, the airflow enters at the upper end and flows out at the upper end, and are not leaked, so that the cigarette cartridge 1 and the atomizing head 2 are easily replaced. In addition, the lower end of the smoke outlet passage 103 is narrower than the upper end, the smoke outlet passage 103 is generally trumpet-shaped with a wide upper portion and a narrow lower portion, which is favorable for forming a negative pressure during smoking, and the smoking effect is better.
2. Two oppositely disposed inserting members 13 are inserted in the smoke outlet passage 103 to reduce the volume of the smoke outlet passage 103, the atomized e-cigarette liquid flows from the gap between the two inserting members 13 to the smoke outlet hole 110, thereby reducing exhaust gas flow and increasing the sensitivity of the sensor 6.
3. The sealing plug 5 is provided with a recess 50 corresponding to the air inlet passage 102. The recess 50 is in communication with the air inlet passage 102. A sensor 6 is mounted in the recess 50, the sensor 6 protrudes downward to form a connecting portion 60, and the connecting portion 60 protrudes from the bottom of the sealing plug 5. The sealing plug 5 can create a negative pressure sensing space for the sensor 6 inside the main body 3, and can also prevent the condensed smoke droplets from falling onto the main board 70 and damaging the main board 70.
4. The bottom side of the main body 10 is provided with a locking hole 109 on two lateral sides thereof. A locking portion 30 is protruded from the two sides of the top of the main body 3, each of the locking portions 30 is correspondingly inserted into one of the locking holes 109. By providing the locking portion 30 on the main body 3, the size of the locking portion 30 can be made larger, thereby effectively preventing the cigarette cartridge 1 from being detached from the control assembly 7.

### Second embodiment

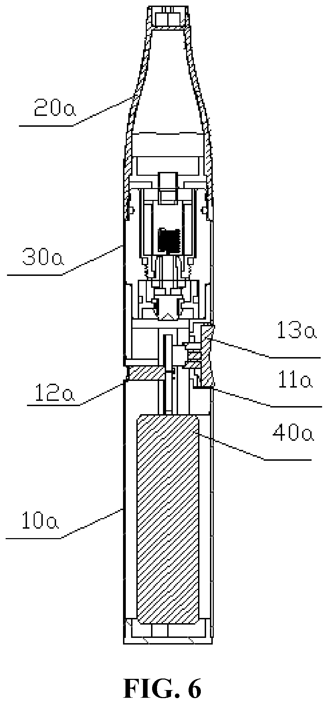

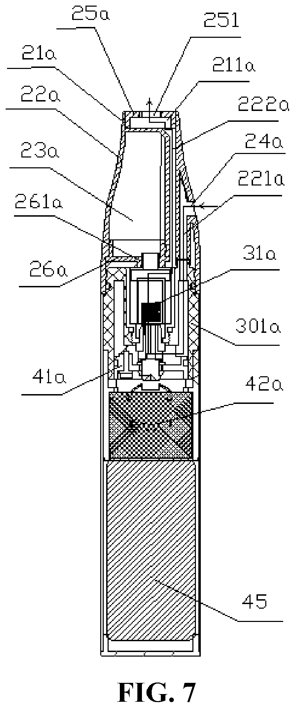

Referring to FIG. 6 and FIG. 7, this embodiment provides an electronic cigarette including a shell 10a, a cigarette cartridge 20a mounted on the top end of the shell 10a, and an atomizing assembly 30a and a control assembly 40a installed in the shell 10a. The cigarette cartridge 20a supplies the e-cigarette liquid to the atomizing assembly 30a, the atomizing assembly 30a atomizes the e-cigarette liquid flowed therein. The control assembly 40a is mounted on the bottom side of the atomizing assembly 30a to supply power to the atomizing assembly 30a and control the operation of the electronic cigarette.

The peripheral wall of the shell 10a is provided with a button hole 11a and a USB port 12a. A trigger button 13a is installed in the button hole 11a. The USB port 12a is configured to charge the battery by an external power source.

The cigarette cartridge 20a has a bottle neck shape, and its bottom end gradually contracts toward the top end. The cigarette cartridge 20a includes a top wall 21a and a peripheral wall 22a. A liquid storage chamber 23a is surrounded by the top wall 21a and the peripheral wall 22a. Further, a cartridge housing (not shown) includes the top wall 21a and the peripheral wall 22a. The top end of the top wall 21a is provided with a receiving groove 211a, the receiving groove 211a is not in communication with the liquid storage chamber 23a. The liquid storage chamber 23a is closed at the top end, the liquid storage chamber 23a has an opening at the bottom end thereof, the opening can be an integral through hole. The thickness of one side wall of the peripheral wall 22a is thicker than the other side wall, it can be considered that the peripheral wall 22a includes a thick wall side and a thin wall side, the peripheral wall is composed of the thick wall side and the thin wall side. Or, the peripheral wall is provided with only one thick wall portion (which can be regarded as the thick wall side), the rest of the peripheral wall is formed by a thin wall potion (which can be regarded as a thin wall side). The thick wall side can be the half circumferential side of the cigarette cartridge 20a. An air inlet passage 221a and a smoke outlet passage 222a are formed on the thick wall side in the axial direction of the cigarette cartridge 20a, that is, the air inlet passage 221a and the smoke outlet passage 222a are formed on the same side of the cigarette cartridge 20a. Further, the cigarette cartridge 20a is provided with a smoke outlet passage 222a and an air inlet passage 221a on the same side thereof, the air inlet passage 221a and the smoke outlet passage 222a are disposed on the same side of the liquid storage chamber 23a. Further, the liquid storage chamber 23a, the smoke outlet passage 222a, and the air inlet passage 221a are arranged closely side-by-side. In one embodiment, a partition wall is formed between the air inlet passage 221a and the smoke outlet passage 222a. The air inlet passage 221a is located adjacent to the outer peripheral surface of the cigarette cartridge 20a, the smoke outlet passage 222a is located adjacent to the liquid storage chamber 23a. An air inlet hole 24a is correspondingly defined on the peripheral wall 22a to communicate with the top end of the air inlet passage 221a, so that after being supplied from the air inlet hole 24a, the airflow can flow out from the bottom end of the peripheral wall along the air inlet passage 221a. The smoke passage 222a extends axially from the bottom end of the peripheral wall 22a to the top end of the peripheral wall 22a, the top thereof is in communication with the receiving groove 211a, so that the airflow enters from the bottom end of the smoke outlet passage 222a and flows out from the top end of the peripheral wall 22a. The top of the smoke outlet passage 222a is narrower than the receiving groove 211a. Further, the receiving groove 211a is provided with a smoke guiding cover 25a for connecting an external cigarette holder (not shown), the center of the smoke guiding cover 25a is provided with a smoke guiding hole 251a corresponding to the center position of the top end of the cigarette cartridge 20a, the airflow flowing into the receiving groove 211a flows out via the smoke guiding hole 251a. The bottom open end of the liquid storage chamber 23a can be further provided with a baffle 26a, the liquid inlet hole 261a is defined in the baffle 26a for the e-cigarette liquid in the liquid storage chamber 23a to flow into the atomizing assembly 30a. The receiving groove 211a can function as a buffer to cool down and prevent hot to the mouth. It can be understood that the peripheral wall of the cigarette cartridge 20a can be integrally provided with a thick wall (regardless of the amount of material or the weight of the cigarette cartridge, etc.). That is, the cigarette cartridge 20a has a sufficient thickness to define the air inlet passage 221a and the smoke outlet passage 222a, and it is only necessary to ensure that the air inlet passage 221a and the smoke outlet passage 222a are defined on the same side of the peripheral wall. By defining the air passage 221 and the smoke outlet passage 222a on the same side of the peripheral wall, the length of the air passage from the air inlet hole to the smoke outlet hole is shortened, thereby effectively solving the problem that the user needs to use a great sucking force to inhale the smoke, the user's inhaling is more convenient and labor-saving.

The atomizing assembly 30a is mounted at the bottom side of the cigarette cartridge 20a, two ends of the atomizing assembly 30a are connected to the outlet of the air inlet passage 221a and the inlet of the smoke outlet passage 222a, respectively. The atomizing assembly 30a atomizes the e-cigarette liquid flowing from the liquid storage chamber of the cigarette cartridge 20a to generate smoke, so that the smoke can be carried out by the airflow. The atomizing assembly 30a includes a main body 301a and an atomizing head 31a installed in the main body 301a. The main body 301a has a tubular shape and is mounted in the shell 10a, the upper end of the main body 301a is tightly coupled to the cigarette cartridge 20a. Optionally, the liquid inlet hole 261a is disposed adjacent to the smoke outlet passage 222a, the atomizing head 31a is disposed below the liquid inlet hole 261a and is in communication with the liquid inlet hole 261a, thereby shortening the distance between the atomizing head 31a and the smoke outlet passage 222a, improving the atomization effect and increasing the amount of smoke.

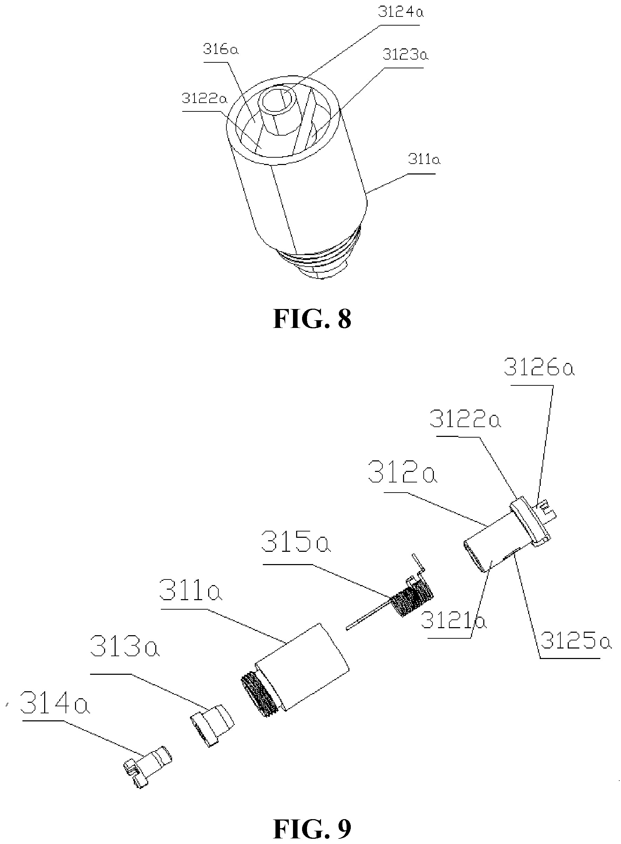

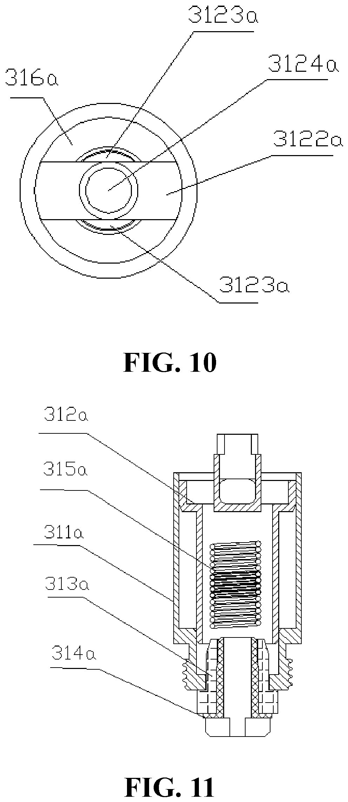

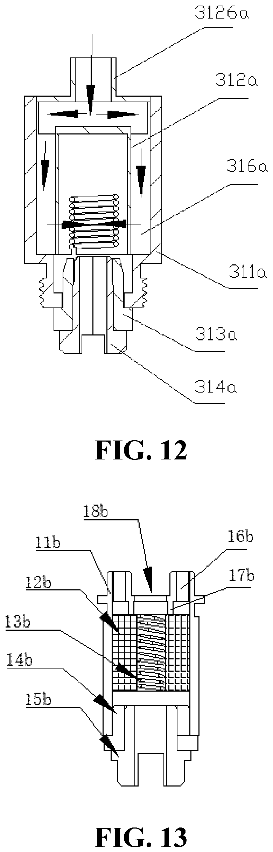

Referring to FIG. 8 and FIG. 9, the atomizing head 31a includes a liquid storage tube 311a, an inner sleeve member 312a, an insulating member 313a, a conductor 314a and a heating member 315a. The liquid storage tube 311a is used for storing the e-cigarette liquid flowing in from the cigarette cartridge and serving as a conductive member at the same time, for example, the atomizing head is electrically connected to one end of an electrode having an opposite polarity relative to the end connected to the conductor 314a. One end of the liquid storage tube 311a is formed as a threaded end for the liquid storage tube 311a to be stably installed in the main body 301a. The inner sleeve member 312a is mounted in a liquid storage tube 311a. The inner sleeve member 312a includes an atomizing tube 3121a and a liquid guiding tube 3122a radially disposed at one end of the atomizing tube 3121a. The interior space of the atomizing tube 3121a is an atomizing heating passage. The liquid guiding tube 3122a is disposed at the top end of the atomizing tube 3121a, both ends of the liquid guiding tube 3122a protrude towards the peripheral wall of the atomizing tube 3121a. The width of the liquid guiding tube 3122a is narrower than the size of the atomizing tube 3121a, such that two smoke outlet holes 3123a are formed at one end of the atomizing tube 3121a and located at two sides of the liquid guiding tube 3122a. The length of the liquid guiding tube 3122a is equal to the inner size of the liquid storage tube 311a, the inner sleeve member 312a is installed in the liquid storage tube 311a, both ends of the liquid guiding tube 3122a are connected to the inner circumferential surface of the liquid storage tube 311a (for example, by screwing connection). A liquid storage gap 316a is formed between the outer circumferential surface of the atomizing tube 3121a and the inner circumferential surface of the liquid storage tube 311a (refer to FIG. 10). Referring to FIG. 11, the atomizing tube 3121a is folded outward at a position flush with the bottom of the liquid guiding tube. After the folding, the atomizing tube 3121a abuts against the liquid storage tube, thereby sealing the top of the liquid storage gap 316a. Referring to FIG. 12, a liquid drop hole (not shown) is respectively disposed on the bottom surfaces of the two ends of the liquid guiding tube 3122a extending from the atomizing tube 3121a to connect the liquid guiding tube 3122a with the liquid storage gap 316a. The top surface of the liquid guiding tube 3122a is provided with a liquid injecting hole 3124a for allowing the e-cigarette liquid to flow into the liquid storage chamber 23a. In the present embodiment, a liquid guiding pipe 3126a is provided at the top end of the liquid guiding tube 3122a, the nozzle of the liquid guiding pipe 3126a is the liquid injecting hole 3124a. Optionally, the liquid guiding pipe 3126a has a circular cross section, the liquid guiding pipe 3126a is in communication with the liquid guiding tube 3122a, the liquid guiding pipe 3126a cooperates with the liquid inlet hole 261a defined at the bottom of the liquid storage chamber 23a for allowing the e-cigarette liquid to flow into the liquid guiding tube 3122a and flow into the liquid storage gap 316a. In addition, a liquid inlet opening 3125a is formed in the pipe wall of the atomizing pipe 3121a, so that the e-cigarette liquid in the liquid storage gap 316a can flow into the atomizing pipe 3121a to be atomized.

The insulating member 313a is sleeved on the conductor 314a and installed at one end of the liquid storage tube 311a, so that the conductive member 314 is insulated from the liquid storage tube 311a. The conductor 314a and the insulating member 313a are respectively provided with a shaft hole (not shown) for introducing airflow from the bottom into the atomizing tube 3121a. The heating member 315a is mounted in the atomizing tube 3121a, one end of the heating member 315a is connected to the liquid storage tube 311a, and the opposite end of the heating member 315a is connected to the conductor 314a. For the liquid storage tube 311a and the conductor 314a, one of them is connected to the positive pole and the other is connected to the negative pole. Further, the circumference of the heating member 315a can be wrapped by a liquid guiding member (not shown), the liquid guiding member wraps around the heating member, and is integrally mounted in the inner cavity of the atomizing tube 3121a. The heating member 315a is selected as a spiral formed by a heating wire, the liquid guiding member is selected from a liquid absorbent cotton or a fiber rope. Further, the heating member may wrap around the liquid guiding member.

After the atomizing head 31a is installed in the main body 301a, the atomizing head 31a is installed at the bottom end of the cigarette cartridge 20a, the top end of the liquid storage tube 311a of the atomizing head 31a is sealed against the bottom surface of the partition wall between the air inlet passage 221a and the air smoke outlet passage 222a at the bottom of the cigarette cartridge 20a. The baffle 26a is sealed against the open end of the bottom of the liquid storage chamber 23a, thereby, the air inlet passage 221a is isolated from the smoke outlet passage 222a. An air gap in communication with the air inlet passage 221a is formed between the outer circumferential surface of the liquid storage tube 311a and the inner circumferential surface of the main body 301a. The air entering the air inlet passage 221a is introduced to the bottom of the atomizing head 31a through the air gap, and is guided into the interior space of the atomizing tube 3121a from the axial hole of the conductive member 314. After the air passes through the interior space of the atomizing tube 3121a, the smoke is taken out from the smoke outlet hole 3123a, and flows out to the smoke outlet passage 222a.

Referring to FIG. 11 and FIG. 12, the e-cigarette liquid flowing out of the liquid storage chamber 23a flows through the liquid guiding tube 3122a into the liquid storage gap 316a, and enters the atomizing tube 3121a via the liquid inlet opening 3125a, the inside of the atomizing tube 3121a is further atomized by the heating member 315a; the airflow of the air inlet passage 221a enters the atomizing heating passage, and the smoke in the atomizing heating passage is taken out through the smoke outlet passage 222a.

The control assembly 40a is responsive to inhaling for supplying power to the atomizing head 31a. The control assembly 40a includes a mounting bracket 41a, a circuit board 42a and a power source 45a that are mounted in the shell 10a. The circuit board 42a is mounted on the mounting bracket 41a. The USB board is provided on the circuit board 42a to match the USB port 12a of the shell 10a for connection to the charger. A switch that matches the trigger button 13a is also disposed on the circuit board 42a to turn the heating member 315a on or off. The power source 45a is detachably mounted in the shell 10a below the circuit board 42a for supplying power to the circuit board 42a and the atomizing head 31a. The power source 45a may be a battery or a battery cell.

When installing the atomizing head of the electronic cigarette, the atomizing head 31a is firstly twisted into the main body 301a, and then the atomizing head 31a is inserted into the through hole at the bottom of the cigarette cartridge 20a; at the same time, the cigarette cartridge 20a and the main body 301a of the atomizing assembly are tightly locked (snap-fitted). When smoking, the trigger button 13a is pressed to smoke normally. When there is no power, it can be charged through the USB port 12a. The electronic cigarette of this embodiment is used as a disposable cigarette, the cigarette cartridge 20a does not have a liquid injection hole, the cigarette cartridge 20a cannot be refilled with e-cigarette liquid. It can be understood that when it is required to be used multiple times, it is required to define a liquid injection hole (not shown) on the cigarette cartridge 20a, for example, a liquid injection hole is radially defined at the peripheral wall 22a, through which the e-cigarette liquid is injected into the liquid storage chamber 23a. The liquid injection hole is not connected to the air inlet passage 221a or the smoke outlet passage 222a, so as to ensure that the e-cigarette liquid can be directly injected into the liquid storage chamber 23a, for example, the liquid injection hole is defined in the peripheral wall 22a and does not in communication with the air inlet passage 221a or the smoke outlet passage 222a.

In summary, the airflow of the electronic cigarette provided in this embodiment enters from the outer periphery of the cigarette cartridge 20a and passes through the atomizing head 31a. The airflow firstly enters the bottom of the atomizing head 31a, then exits from above the atomizing head 31a, and finally, the airflow is vented through the top end of the cigarette cartridge 20a, to complete a suck. The air inlet passage 221a and the smoke outlet passage 222a are disposed on the same side, the airflow enters at the upper end and flows out at the upper end, and the e-cigarette liquid are not leaked. Meanwhile, the cigarette cartridge 20a and the atomizing head 31a are easily replaced.

### Third embodiment

As shown in FIGs. 13-18, this embodiment discloses an electronic cigarette including a shell 40b, a battery assembly 30b and an atomizing assembly. The battery assembly 30b is disposed in the shell 40b for supplying power to the atomizing assembly. The atomizing assembly includes a cigarette cartridge 21b and an atomizer. The cigarette cartridge 21b is mounted on the upper end of the shell 40b. The atomizer is detachably fixed to the bottom end of the cigarette cartridge 21b and disposed within the shell 40b.

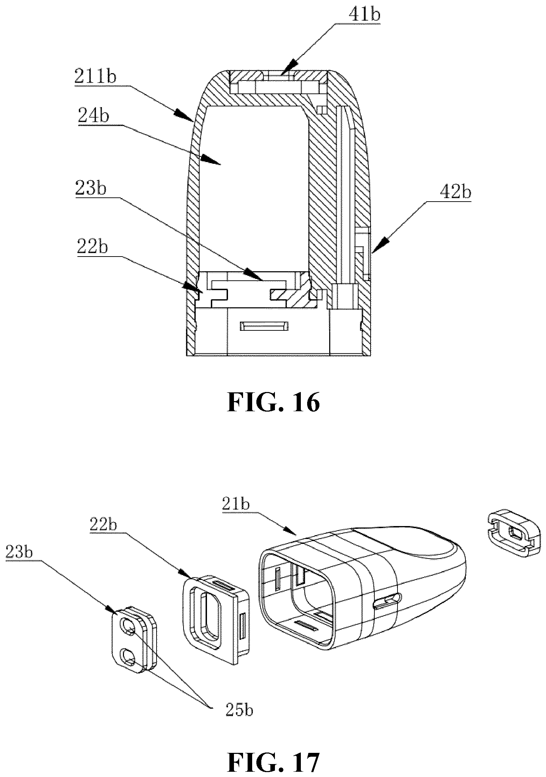

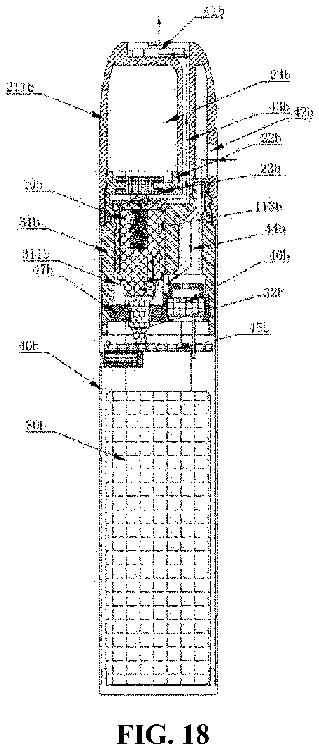

As shown in FIG. 16 and FIG. 18, the cigarette cartridge 21b includes a cartridge housing 211b, the cartridge housing 211b has a liquid storage chamber 24b and a smoke outlet passage 43b. The bottom end of the liquid storage chamber 24b is provided with a liquid storage chamber hole 25b. The top end of the cartridge housing 211b is provided with a smoke outlet hole 41b, and one end of the smoke outlet passage 43b is in communication with the smoke outlet hole 41b.

As shown in FIG. 18, the atomizer includes an atomizing head main body 31b and an atomizing head 10b. The atomizing head main body 31b is partially received at the bottom end of the cartridge housing 211b and fixedly connected to the cartridge housing 211b, the remaining of the atomizing head main body 31b is partially disposed in the shell 40b. The atomizing head main body 31b is provided with an atomizing head mounting hole 311b, the atomizing head 10b is disposed in the atomizing head mounting hole 311b. In one embodiment, the bottom of the liquid storage chamber 24b is provided with a sealing member 23b, the liquid storage chamber hole 25b is disposed on the sealing member 23b. Specifically, the sealing member 23b is a silicone gasket or a rubber gasket.

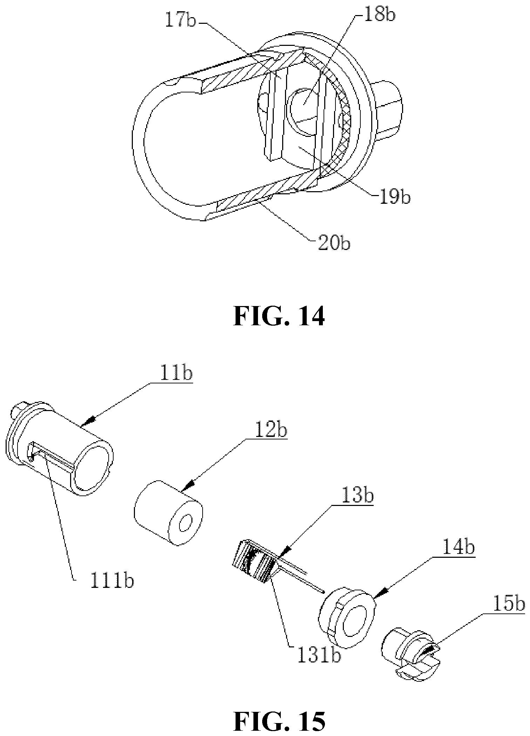

As shown in FIG. 1 and FIG. 2, the atomizing head 10b includes an atomizing head housing 11b, a heating member 13b, and a liquid guiding member 12b. The atomizing head housing 11b includes a housing body 20b and an atomizing head top cover 19b fixedly connected to the upper end of the housing body 20b. A cavity is formed inside the housing body 20b, the heating member 13b and the liquid guiding member 12b are both located in the cavity. The liquid guiding member 12b is sleeved on the outer circumference of the heating member 13b. The atomizing head top cover 19b is provided with a liquid inlet hole 16b in communication with the cavity. The liquid guiding member 12b is located under the liquid inlet hole 16b to ensure that the e-cigarette liquid flowing through the liquid inlet hole 16b can be guided into the liquid guiding member 12b. A blocking portion 17b is disposed between the heating member 13b and the liquid inlet hole 16b. The upper end surface of the blocking portion 17b abuts against the lower end surface of the atomizing head top cover 19b, the lower end surface of the blocking portion 17b abuts against the upper end surface of the liquid guiding member 12b. Alternatively, the blocking portion 17b is integrally formed with the atomizing head housing 11b. The liquid inlet hole 16b is in communication with the liquid storage chamber hole 25b at the bottom end of the liquid storage chamber 24b. It should be noted that the function of the blocking portion 17b is to block the direct flow of the e-cigarette liquid flowing from the liquid inlet hole 16b to the heating member 13b, so that the e-cigarette liquid can only flow to the liquid guiding member 12b, and then is conducted to the surface of the heating member 13b through the liquid guiding member 12b. Therefore, the arrangement of the blocking portion 17b can be as shown in FIG. 14, that is, including two strip-shaped blocking portions 17b. It can be understood that when the liquid inlet hole 16b includes more than two (excluding two), the corresponding number of the blocking portions 17b can be correspondingly set according to the above setting manner. Alternatively, more than two (excluding two) of the above-mentioned liquid inlet holes 16b are respectively disposed on both sides of the two blocking portions 17b. In one embodiment, the blocking portion 17b is an annular body, and disposed around the top end of the heating member 13b, at this time, the liquid inlet hole 16b can be any number and are disposed outside of the blocking portion 17b. Alternatively, the blocking portion 17b may have a closed shape such as a quadrangle or a pentagon. Further, in one embodiment, the blocking portion 17b extends downward in the axial direction of the liquid inlet hole 16b, and the lower end is formed to abut against the liquid guiding member 12b, at this time, the liquid inlet hole 16b can be any number, the shape of the blocking portion 17b depends on the shape of the liquid inlet hole 16b, the blocking portion 17b is disposed around the liquid inlet hole 16b, as long as the e-cigarette liquid flowing through the liquid inlet hole 16b flows through the liquid guiding member 12b and then contacts the heating member 13b. In one embodiment, the blocking portion 17b is a polygonal shape that acts as a sealing member.

As shown in FIGs. 13-15 and 17-18, an air outlet hole 18b is further disposed on the atomizing head top cover 19b of the atomizing head, an atomizing head air passage 131b is formed inside the heating member 13b, the atomizing head air passage 131b is in communication with the outside air. Specifically, one end of the atomizing head air passage 131b is connected to the air outlet hole 18b, the air outlet hole 18b is in communication with the smoke outlet passage 43b and the smoke outlet hole 41b of the cartridge housing 211b.

As shown in FIG. 18, the cigarette cartridge 21b is also connected with the atomizing head main body 31b to form an air inlet passage 44b. An air inlet hole 42b in communication with the air inlet passage 44b is provided on an outer peripheral wall of the cartridge housing 211b, the cigarette cartridge 21b is formed on the same side with a smoke outlet passage 43b and an air inlet passage (not shown). The air inlet passage and the smoke outlet passage 43b are disposed on the same side of the liquid storage chamber 24b.

Further, the liquid storage chamber 24b, the smoke outlet passage 43b and the air inlet passage are arranged closely side-by-side. The air inlet hole 42b is in communication with the air inlet passage, the other end of the atomizing head air passage 131b is in communication with the air inlet passage 44b.

A matching portion 113 is disposed in the atomizing head mounting hole 311b. The atomizing head 10b is correspondingly provided with a limiting portion 111. The atomizing head 10b is fixed in the atomizing head mounting hole 311b by the matching portion 113. Specifically, the limiting portion 111 is an L-shaped groove formed on the outer peripheral wall of the housing body 20b; and corresponding to the position of the L-shaped groove, the inner peripheral wall of the atomizing head mounting hole 311b is provided with a protrusion that cooperates with the L-shaped groove, the protrusion is the matching portion 113. Alternatively, the limiting portion 111 is a protrusion formed on the outer peripheral wall of the housing body 20b; and corresponding to the position of the protrusion, the inner peripheral wall of the atomizing head mounting hole 311b is provided with an L-shaped groove that cooperates with the protrusion, the L-shaped groove is the matching portion 113. The atomizing head 10b in this embodiment is mounted and disassembled as follows: one end of the atomizing head 10b is caused to align with the atomizing head mounting hole 311b, and the protrusion is caused to align with the entrance of the L-shaped groove. At this time, the atomizing head 10b can be pushed into the atomizing head mounting hole 311b, the protrusion moves along the L-shaped groove until reaching the bottom end of the groove, then the atomizing head 10b can be rotated to a certain mounting angle. Therefore, the matching structure of the above-mentioned protrusion and the L-shaped groove has a guiding function and a positioning function.

As shown in FIG. 18, the electronic cigarette further includes a sensor 46b. A sealing gasket 47b is disposed between the battery assembly 30b and the atomizing assembly. The sealing gasket 47b is fixed to the bottom end of the atomizing head main body 31b. The sensor 46b is fixed in the sealing gasket 47b, the sensor 46b is in communication with the air inlet passage 44b and is electrically connected to the battery assembly 30b.

Further, the electronic cigarette further includes a circuit board 45b. The circuit board 45b is disposed between the atomizer and the battery assembly 30b. In one embodiment, the sensor 46b, the atomizer and the battery assembly 30b are all electrically connected to the circuit board 45b. The sensor 46b, the atomizer, and the battery assembly 30b realize circuit connection and signal transmission through the circuit board 45b.

Optionally, the sensor 46b is a pressure sensing sensor. The air passage of the pressure sensing sensor is in communication with the air inlet passage 44b. Therefore, when the air inlet passage 44b supplies air into the atomizer 11, the gas in the air passage of the pressure sensing sensor is taken away, causing a negative pressure, and the pressure sensing sensor generates an electric signal and transmits the electric signal to the circuit board 45b. The battery assembly 30b includes a battery (not shown). The circuit board 45b controls the battery to supply power, so that the electronic cigarette operates.

In the electronic cigarette of this embodiment, the liquid storage chamber 24b is configured for storing the e-cigarette liquid, and the e-cigarette liquid flows into the atomizing head 10b via the liquid storage chamber hole 25b and the liquid inlet hole 16b. In the atomizing head 10b, the liquid inlet hole 16b is directly aligned with the liquid guiding member 12b and is further provided with a blocking portion 17b. Therefore, the e-cigarette liquid must be infiltrated from the liquid guiding member 12b to the heating member 13b, avoiding the technical problem that the heating member 13b is immersed by the e-cigarette liquid. In addition, a sealing gasket 47b is provided between the battery assembly 30b and the atomizing assembly, the sealing gasket 47b is also located between the circuit board 45b and the atomizer, to prevent the condensate and the e-cigarette liquid from falling onto the circuit board 45b to cause the circuit board 45b to be damaged.

The above-mentioned embodiments merely represent several implementations of the present application, and the descriptions thereof are more specific and detailed, but they shall not be understood as a limitation on the scope of the present application. It should be noted that, for those of ordinary skill in the art, variations and improvements may still be made without departing from the concept of the present application, and all of which shall fall into the protection scope of the present application. Therefore, the scope of protection of the present application shall be subject to the appended claims.

## Claims

1. A cigarette cartridge comprising a cartridge housing, a liquid storage chamber, an air inlet passage and a smoke outlet passage, wherein the liquid storage chamber, the air inlet passage and the smoke outlet passage are provided in the cartridge housing, the air inlet passage and the smoke outlet passage are disposed on the same side of the liquid storage chamber.

2. The cigarette cartridge according to claim **1,** wherein the liquid storage chamber, the air inlet passage and the smoke outlet passage are arranged closely side-by-side.

3. The cigarette cartridge according to claim **1,** wherein the cigarette cartridge comprises a top wall and a peripheral wall, the liquid storage chamber is surrounded by the top wall and the peripheral wall, the air inlet passage and the smoke outlet passage are provided in the peripheral wall and located on the same side of the cigarette cartridge.

4. The cigarette cartridge according to claim **2,** wherein an air inlet hole is defined in the peripheral wall of the cigarette cartridge, the air inlet hole is in communication with the air inlet passage.

5. The cigarette cartridge according to claim **1,** wherein a partition wall is formed between the air inlet passage and the smoke outlet passage.

6. The cigarette cartridge according to claim **1,** wherein the lower end of the smoke outlet passage is narrower than the upper end of the smoke outlet passage.

7. The cigarette cartridge according to claim **1,** wherein an inserting member is disposed in the smoke outlet passage, after the inserting member is inserted into the smoke outlet passage, the smoke outlet passage is substantially trumpet-shaped having a wide upper end and a narrow lower end.

8. The cigarette cartridge according to claim **1,** wherein the cigarette cartridge comprises a main body, a top cover and a bottom cover, the top cover is provided with a smoke outlet hole, the smoke outlet passage is in communication with the smoke outlet hole.

9. The cigarette cartridge according to claim **1,** wherein the inner wall of the smoke outlet passage is provided with a slope or a bump, after the smoke outlet passage is provided with the slope or the bump, the smoke outlet passage is substantially trumpet-shaped having a wide upper end and a narrow lower end.

10. The cigarette cartridge according to claim **7,** wherein the inserting member has a wedge-shaped upper end and a column-shaped lower end, or the inserting member is a cylinder, the outer wall of the cylinder is axially attached to the inner wall of the smoke outlet passage, and a tapered hole is defined in the inserting member and penetrates through the inserting member, the size of the tapered hole gradually decreases from top to bottom.

11. An atomizing assembly comprising the cigarette cartridge according to any one of claims **1** to **10,** the atomizing assembly further comprising an atomizer detachably secured to the bottom end of the cigarette cartridge.

12. The atomizing assembly according to claim **11,** wherein the atomizer comprises an atomizing head, the atomizing head comprises an atomizing head housing, a heating member and a liquid guiding member, the atomizing head housing comprises a housing body and an atomizing head top cover, a cavity is defined inside the housing body, the heating member and the liquid guiding member are both located in the cavity, the liquid guiding member is sleeved on the outer circumference of the heating member, the atomizing head top cover is provided with a liquid inlet hole in communication with the cavity, the liquid guiding member is located under the liquid inlet hole to ensure the e-cigarette liquid flowing through the liquid inlet hole to be guided into the liquid guiding member, a blocking portion is disposed between the heating member and the liquid inlet hole, the upper end surface of the blocking portion abuts against the lower end surface of the atomizing head top cover, the lower end surface of the blocking portion abuts against the upper end surface of the liquid guiding member to prevent the e-cigarette liquid flowing through the liquid inlet hole from directly contacting the heating member without flowing through the liquid guiding member.

13. The atomizing assembly according to claim **12,** wherein the atomizer comprises an atomizing head main body, the atomizing head main body is provided with an atomizing head mounting hole, the atomizing head is disposed in the atomizing head mounting hole.

14. The atomizing assembly according to claim **13,** wherein the outer peripheral wall of the housing body is provided with a limiting portion.

15. The atomizing assembly according to claim **14,** wherein the atomizing head mounting hole is provided with a matching portion that is fixedly coupled with the limiting portion, the atomizing head is fixed in the atomizing head mounting hole by the matching portion.

16. The atomizing assembly according to claim **15,** wherein the limiting portion is an L-shaped groove provided in the outer peripheral wall of the housing body; corresponding to the position of the L-shaped groove, the inner peripheral wall of the atomizing head mounting hole is provided with a protrusion that cooperates with the L-shaped groove, the protrusion is the matching portion; or, the limiting portion is a protrusion formed on the outer peripheral wall of the housing body; corresponding to the position of the protrusion, the inner peripheral wall of the atomizing head mounting hole is provided with an L-shaped groove that cooperates with the protrusion, the L-shaped groove is the matching portion.

17. An atomizing assembly comprising the cigarette cartridge according to any one of claims **1** to **10,** the atomizing assembly further comprising an atomizer, the atomizer being provided with an atomizing heating passage, one end of the atomizing heating passage is in communication with the air inlet passage, the other end is in communication with the smoke outlet passage.

18. The atomizing assembly according to claim **17,** wherein the atomizing head comprises a liquid storage tube and an inner sleeve member inserted into the liquid storage tube, the inner sleeve member comprises an atomizing tube, the interior space of the atomizing tube is the atomizing heating passage.

19. The atomizing assembly according to claim **18,** wherein the atomizing head comprises a heating member and a liquid guiding member, the heating member is wrapped around the liquid guiding member, or the liquid guiding member is wrapped around the heating member.

20. An electronic cigarette comprising the atomizing assembly according to any one of claims **11** to **19.**
